# EUROPÄISCHE PATENTSCHRIFT

(11) **EP 0 708 650 B1**
(45) Veröffentlichungstag und Bekanntmachung des Hinweises auf die Patenterteilung: **17.12.1997**
(21) Anmeldenummer: 93915881.2
(22) Anmeldetag: 12.07.1993
(51) Int. Cl.: A61K 35/78, A61K 7/48, A61K 7/06, B01D 11/02

(54) **VERFAHREN ZUR HERSTELLUNG FLÜSSIGER PFLANZENAUSZÜGE**
METHOD OF PREPARING LIQUID PLANT EXTRACTS
PROCEDE DE PRODUCTION D'EXTRAITS LIQUIDES DE PLANTES

(43) Veröffentlichungstag der Anmeldung: 01.05.1996
(73) Patentinhaber: M.C.M. KLOSTERFRAU VERTRIEBSGESELLSCHAFT m.b.H., 50670 Köln (DE)
(72) Erfinder: GREVE, Rainer, D-23795 Bad Segeberg (DE); PFADT, Joachim, D-23795 Gross Rönnau (DE); KINDLUND, Birgit, D-24539 Neumünster (DE)
(74) Vertreter: von Raffay, Vincenz, Dipl.-Ing.
(86) Internationale Anmeldenummer: EP9301819
(87) Internationale Veröffentlichungsnummer: WO9502410

(56) Entgegenhaltungen:
- DE-C- 4 205 783
- DATABASE WPI Week 8617, Derwent Publications Ltd., London, GB; AN 86-109205 & JP,A,61 050 920 (ICHIMARU FALCOS KK) 13. März 1986
- DATABASE WPI Week 7814, Derwent Publications Ltd., London, GB; AN 78-26120 & JP,A,53 018 715 (ISHIHARA H.) 21. Februar 1978

## Beschreibung

Die vorliegende Erfindung betrifft ein Verfahren zur Herstellung von flüssigen Pflanzenauszügen, das ohne den Einsatz von Ethanol auskommt.

Flüssige Pflanzenauszüge sind Zubereitungen aus zerkleinerten, frischen oder getrockneten Pflanzen oder Pflanzenteilen (Drogen), die durch Extraktion mit geeigneten Flüssigkeiten gewonnen werden.

Es handelt sich um vielverwendete, je nach Herkunft und Gewinnungsart unterschiedlich zusammengesetzte Produkte, die z. B. zur Herstellung von Lebens- und Genußmitteln, flüssigen Würzen, Duft- und Aromamischungen oder Arzneimitteln eingesetzt werden. Dabei können sie als Ausgangsstoffe für die Weiterverarbeitung zu Fertigprodukten oder auch direkt als solche verwendet werden; bei Arzneimitteln einerseits in Vermischung mit Hilfs- und Trägerstoffen z. B. zur Herstellung von Sirupen, Säften, Cremes, Salben oder Badezusätzen, andererseits unvermischt als konzentrierte Tropflösung oder als Teeaufguß.

In der pharmazeutischen Terminologie unterscheidet man bei flüssigen Pflanzenauszügen je nach Art des Auszugsmittels, der Herstellungsweise und der Konzentration (Droge-Extrakt-Verhältnis, DEV) zwischen Aufgüssen, Abkochungen, Tinkturen und Fluidextrakten.

Aufgüsse und Abkochungen werden in der Siedehitze mit Wasser als Auszugsmittel hergestellt. Sie sind für den sofortigen Verbrauch bestimmt und haben wegen mangelhafter Haltbarkeit bei der Herstellung von lagerstabilen Fertigarzneimitteln keine Bedeutung.

Pharmazeutische Tinkturen und Fluidextrakte werden nach den anerkannten Regeln des Deutschen Arzneibuches (DAB) oder anderer Pharmakopöen hergestellt. Als Auszugsflüssigkeit werden fast ausschließlich Ethanol oder Mischungen von Ethanol und Wasser, gegebenenfalls mit bestimmten Zusätzen, verwendet. Die Herstellung erfolgt in aller Regel bei Raumtemperatur nach den an sich bekannten Verfahren der Mazeration oder der Perkolation, gegebenenfalls auch der Reperkolation, in der Weise, daß Tinkturen aus einem Teil Droge und fünf bis zehn Teilen Extraktionsflüssigkeit gewonnen werden; bei Fluidextrakten werden aus einem Teil Droge höchstens zwei Teile Produkt hergestellt, so daß hier eine relativ hohe Konzentration pflanzlicher Inhaltsstoffe vorliegt.

Tinkturen und Fluidextrakte weisen wegen ihres Ethanolgehaltes, der meistens zwischen 30 und 70 Vol.%, manchmal auch bis 90 Vol.% betragen kann, gegenüber wäßrigen Auszügen einige Vorzüge auf. Sie enthalten ein breites Inhaltsstoffspektrum sowohl lipophiler als auch hydrophiler Komponenten, sind weitgehend lagerstabil und relativ unempfindlich gegen Luftsauerstoff und Verkeimung. Die überwiegende Mehrzahl aller heute bekannten, flüssigen oder halbfesten pflanzlichen Arzneimittel, z. B. Säfte, Sirupe, Tropfen, Emulsionen oder Salben, werden daher aus Tinkturen oder, bevorzugt, aus Fluidextrakten hergestellt. Aus diesem Sachverhalt ergibt sich zwangsläufig, daß alle flüssigen oder halbfesten pflanzlichen Arzneimittel, die auf der Basis von Tinkturen oder Fluidextrakten hergestellt werden, einen mehr oder weniger hohen Gehalt an Ethanol aufweisen.

Aus heutiger medizinischer Sicht besteht aber angesichts bestimmter Patientengruppen, z. B. bei Kindern aller Altersstufen, Alkoholikern, Schwangeren oder Leberkranken, ein dringendes Bedürfnis nach alkoholfreien Phytopharmaka, besonders wenn diese innerlich (oral) verabreicht werden sollen. Aufgabe der vorliegenden Erfindung ist es deshalb, ethanolfreie flüssige Pflanzenauszüge herzustellen.

Theoretisch könnte den Tinkturen bzw. Fluidextrakten, bevor sie zur Herstellung von Arzneimitteln verwendet werden, das Ethanol mittels Vakuumdestillation entzogen werden. Dem steht aber entgegen, daß bei einer großen Zahl von Pflanzen die wert- und wirksamkeitsbestimmenden Inhaltsstoffe flüchtig sind (z. B. ätherische Öle), so daß sie während der Destillation mit dem Ethanol übergehen und von diesem nicht mehr zu trennen sind. Außerdem wird bei allen ethanolhaltigen Extrakten durch den Entzug des Ethanols in dem sehr komplex gelösten Inhaltsstoffgemisch das Gleichgewicht empfindlich gestört, so daß massive, irreversible Fällungen auftreten. Dadurch werden die Extrakte denaturiert und sind für den pharmazeutisch-medizinischen Einsatz nicht mehr brauchbar.

Eine weitere denkbare Möglichkeit, Ethanol auszuschließen, besteht darin, diesen durch andere organisch-chemische Extraktionsmedien mit ähnlichen Eigenschaften zu ersetzen. Eine Lösung zeichnet sich aber nicht ab, weil keine organische Verbindung bekannt ist, die alle günstigen Eigenschaften des Ethanols, und nur diese, auf sich vereinigt. Die meisten organischen Flüssigkeiten sind mehr oder weniger stark toxisch, weisen auch in den meisten Fällen üblen Geruch oder Geschmack auf. Damit sind sie vor allem für die orale Applikation vollkommen ungeeignet.

Das Ausweichen auf wäßrige Extraktionsmedien, die Beimengungen höherer Alkohole enthalten, z. B. Propylenglycol, Glycerol, Mannitol oder Sorbitol, führt hinsichtlich der Inhaltsstoffspektren und -gehalte zu unbefriedigenden Extraktionsergebnissen, wenn man diese mit herkömmlichen ethanolisch-wäßrigen Auszügen vergleicht.

Die Aufgabe vorliegender Erfindung wird nun überraschend dadurch gelöst, daß für die Extraktion Wasser mit einem verhältnismäßig geringen Zusatz von einem oder mehreren nichtionischen Tensiden eingesetzt wird. Dieses Verfahren liefert ethanolfreie Extrakte, die sich qualitativ und quantitativ von alkoholisch-wäßrigen Auszügen kaum noch unterscheiden. Insbesondere enthalten sie in genügender Menge auch diejenigen, überwiegend lipophilen Inhaltsstoffe, wie vor allem ätherische Öle, aber auch Fette, Harzanteile, Flavonoide oder Saponine, die bei einer rein wäßrigen Extraktion auch bei Anwendung von Wärme (Teeaufguß) nicht oder nur wenig in die Wasserphase übergehen.

Die vorliegende Erfindung betrifft demnach ein Verfahren zur Herstellung flüssiger Pflanzenauszüge, dadurch gekennzeichnet, daß als Extraktionsflüssigkeit eine wäßrige Lösung eines oder mehrerer nichtionischer Tenside eingesetzt wird.

Als nichtionische Tenside kommen prinzipiell alle Verbindungen dieser Art in Frage, die pharmazeutisch unbedenklich, d. h. vor allem untoxisch sind. Soll der erfindungsgemäß hergestellte Pflanzenauszug zur Herstellung einer oralen Arzneiform verwendet werden, sollte das verwendete Tensid darüberhinaus möglichst geschmacksneutral sein.

Geeignete nichtionische Tenside sind beispielsweise Fettalkoholpolyethylenglykolether; Alkylphenolpolyethylenglykolether; Monofettsäurepolyethylenglykolester; Fettsäureester von Polyhydroxyverbindungen, wie Glycerin, Diglycerin, Polyglycerin, Erythrit, Pentaerythrit, Xylit, Sorbit, Mannit oder Saccharose, wobei die Polyhydroxyverbindung zusätzlich auch noch mit Polyethylenglykol verethert sein kann; sowie Glykoside von Fettalkoholen. Bevorzugte nichtionische Tenside sind Fettsäureglycerinpolyethylenglykolester.

Ein besonders bevorzugtes nichtionisches Tensid ist Macrogol-Glycerol-hydroxystearat gemäß DAB 9 (Umsetzungsprodukt aus 1 mol hydriertem Rizinusöl mit 40 - 45 mol Ethylenoxid), das auch aufgrund seiner Geschmacks- und Geruchsneutralität besonders vorteilhaft ist.

Die nichtionischen Tenside werden bevorzugt in Mengen von 1 bis 10 Gew%, besonders bevorzugt in Mengen von 5 bis 8 Gew%, jeweils bezogen auf die Extraktionsflüssigkeit, eingesetzt.

Wird eine Mischung von mehreren nichtionischen Tensiden verwendet, so ist deren Gewichtsverhältnis untereinander völlig unkritisch und richtet sich nach den praktischen Erfordernissen. Gewöhnlich bewegt sich der Anteil eines Tensids zwischen 1 und 99 Gew%, bezogen auf die Gesamttensidmenge.

Das erfindungsgemäße Verfahren wird in an sich bekannter Weise, beispielsweise als Mazeration, Perkolation oder auch Reperkolation analog der im Deutschen Arzneibuch oder anderen Pharmakopöen für die Extraktion mit Ethanol-Wasser-Gemischen angegebenen Verfahrensweise ausgeführt.

Die Temperatur liegt dabei vorteilhafterweise zwischen Raumtemperatur und einer solchen kurz unterhalb der Siedetemperatur der Extraktionsflüssigkeit. Bevorzugt liegt sie zwischen 90 und 98 ^{o}C.

Die Vorteile des erfindungsgemäßen Verfahrens gegenüber dem Verfahren des Standes der Technik unter Verwendung von Ethanol-Wasser-Gemischen wird in den Versuchsreihen der folgenden Beispiele dokumentiert:

### Beispiel 1

### a) Herstellung von Thymianextrakten nach erfindungsgemäßem Verfahren bei 95 ^{o}C:

In einem verschließbaren gläsernen Laborkolben werden 20 g getrocknetes Thymiankraut DAB 9 mit 100 g einer auf 95 ^{o}C erhitzten wäßrigen Lösung eines handelsüblichen Macrogol-Glycerolhydroxystearats in der jeweiligen Konzentration übergossen. Zum Schutz vor mikrobiellem Befall werden 0,3 Gew% Kaliumsorbat zugegeben. Man läßt den verschlossenen Ansatz 5 Stunden bei Raumtemperatur stehen, gießt dann die Flüssigkeit ab, preßt den Drogenrückstand aus und filtriert die vereinigten Extraktanteile durch ein Papierfilter.

Zum Vergleich der nach vorstehender Arbeitsvorschrift erhaltenen Extrakte wurde anschließend jeweils der Thymolgehalt photometrisch nach DAB 9 bestimmt. Der Thymolgehalt steht in direktem Verhältnis zum Gehalt an ätherischem Gesamtöl.

### b) Herstellung von Thymianextrakten nach erfindungsgemäßem Verfahren bei Raumtemperatur:

In einem verschließbaren gläsernen Laborkolben werden 20 g getrocknetes Thymiankraut DAB 9 mit 110 g einer auf Raumtemperatur (RT) gehaltenen wäßrigen Lösung eines handelsüblichen Macrogol-Glycerolhydroxystearats in der jeweiligen Konzentration übergossen. Zum Schutz vor mikrobiellem Befall werden 0,3 Gew% Kaliumsorbat zugesetzt. Man läßt den verschlossenen Ansatz 5 Tage unter häufigem Umschütteln bei RT stehen, gießt die überstehende Flüssigkeit ab, preßt den Drogenrückstand aus und gibt die vereinigten Extraktanteile durch ein Papierfilter. Wie unter a) angegeben, wurde von allen Extrakten der Thymolgehalt bestimmt.

### c) Herstellung von Thymianextrakten nach Stand der Technik:

In einem verschließbaren gläsernen Laborkolben werden 20 g getrocknetes Thymiankraut DAB 9 mit 100 g einer Ethanol-Wasser-Mischung des jeweiligen Gehaltes bei Raumtemperatur übergossen. Man läßt 5 Tage unter öfterem Umschütteln stehen, gießt die Flüssigkeit ab, preßt den Drogenrückstand aus und gibt die vereinigten Flüssigkeiten durch ein Papierfilter.

Wie unter a) angegeben, wurde von allen Extrakten der Thymolgehalt bestimmt.

Die folgenden Tabellen zeigen die Ergebnisse der Versuchsreihen:

### erfindungsgemäß

| Tensid-Konzentration (Gew%) | 95 ^{o}C/5 Stunden Thymolgehalt (mg/100 g) | Raumtemperatur/5 Tage Thymolgehalt (mg/100 g) |
|---|---|---|
| 0 | 3,9 | |
| 1 | 12,2 | 11,7 |
| 2 | 21,1 | 21,1 |
| 3 | 33,6 | 27,2 |
| 4 | 37,8 | 33,4 |
| 5 | 42,2 | 38,5 |
| 6 | 46,5 | 42,2 |
| 7 | 45,3 | 41,0 |

### gemäß Stand der Technik

| Ethanol-Konzentration (Gew%) | Raumtemperatur/5 Tage Thymolgehalt (mg/100 g) |
|---|---|
| 10 | 5,5 |
| 20 | 6,9 |
| 30 | 12,9 |
| 40 | 26,1 |
| 50 | 43,3 |
| 60 | 45,1 |
| 70 | 44,2 |

Ein Vergleich des erfindungsgemäßen Verfahrens bei Raumtemperatur mit dem Verfahren gemäß Stand der Technik zeigt, daß die wäßrigen Tensidlösungen jeweils dieselbe Extraktionswirkung besitzen wie zehn-bis zwanzigfach höhere Ethanolkonzentrationen. Die optimale Extraktionswirkung wird bei einer 6 %igen Tensidlösung bzw. bei einer 60 %igen Ethanollösung erreicht.

Ein Vergleich des erfindungsgemäßen Verfahrens bei Raumtemperatur/5 Tage einerseits und bei 95 ^{o}C/5 Stunden andererseits ergibt im Thymolgehalt keinen signifikanten Unterschied. Die Heißmazeration hat aber den unübersehbaren Vorteil einer großen Zeitersparnis. Weiterhin ist angesichts der starken Verkeimung aller natürlichen Drogen die keimtötende Wirkung der heißen Tensidlösung ein erwünschter Effekt, der in der Kälte nur mit Ethanol zu erreichen ist.

### Beispiel 2

Die Verfahren gemäß Beispiel 1a) und 1b) wurden wiederholt, mit dem Unterschied, daß als Tensid eine Mischung aus Macrogol-1500-glyceroltriricinoleat DAC (Tensid 1) und Polysorbat 20 DAB 9 (Tensid 2) eingesetzt wurde. Der Gesamttensidanteil betrug jeweils 6 Gew%. Die folgende Tabelle zeigt die Ergebnisse:

| Tensid 1 (Gew%) | Tensid 2 (Gew%) | Thymolgehalt (mg/100 g) | |
|---|---|---|---|
| | | 95 ^{o}C | Raumtemperatur |
| 1 | 5 | 42,2 | 39,8 |
| 2 | 4 | 41,8 | 40,2 |
| 3 | 3 | 42,8 | 39,7 |
| 4 | 2 | 42,1 | 40,5 |
| 5 | 1 | 41,0 | 39,6 |

### Beispiel 3

Das Verfahren gemäß Beispiel 1a) wurde mit jeweils 6 %igen Tensidlösungen wiederholt, wobei aber anstelle von Thymiankraut Pfefferminzblätter eingesetzt wurden. Als Tenside wurden Macrogol-1500-glyceroltriricinoleat DAC (Tensid 1), Polysorbat 20 DAB 9 (Tensid 2) und Macrogol-Glycerolhydroxystearat (Tensid 3) verwendet.

Zum Vergleich zum Stand der Technik wurden Pfefferminzblätter auch gemäß Beispiel 1c) mit 60 Gew%igem Ethanol extrahiert.

Die Extraktionsergebnisse wurden durch gaschromatographische Bestimmung des Mentholgehaltes der Extrakte gegen eine Menthol-Vergleichslösung bestimmt. Die Mentholausbeute steht in direktem Verhältnis zur Asubeute an Gesamtöl.

| Tensid | Mentholgehalt (mg/100 g) |
|---|---|
| 1 | 285 |
| 2 | 293 |
| 3 | 310 |

Vergleich:
Ethanol 60 Gew% 295

### Beispiel 4

Beispiel 3 wurde wiederholt, wobei anstelle von Pfefferminzblättern Eucalyptusblätter verwendet wurden. Die Extraktionsergebnisse wurden durch gaschromatographische Bestimmung des Cineolgehaltes der Extrakte bestimmt.

| Tensid | Cineolgehalt (mg/100 g) |
|---|---|
| 1 | 1185 |
| 2 | 1119 |
| 3 | 1254 |

Vergleich:
Ethanol 60 Gew% 1210

### Beispiel 5

Die Verfahren gemaß Beispiel 1a) und 1b) wurden wiederholt mit dem Unterschied, daß als Tensid eine handelsübliche Lauryl-Polyglycose eingesetzt wurde. Die folgende Tabelle zeigt die Ergebnisse:

| Tensid-konzentration (Gew.%) | 95°C/5 h Thymolgehalt (mg/100 g) | Raumtemp./5 Tage Thymolgehalt (mg/100 g) |
|---|---|---|
| 0 | 4,1 | |
| 1 | 13,2 | 11,9 |
| 2 | 22,3 | 21,0 |
| 3 | 34,8 | 31,6 |
| 4 | 38,5 | 36,7 |
| 5 | 44,7 | 42,1 |
| 6 | 45,2 | 42,4 |
| 7 | 45,1 | 42,8 |

## Patentansprüche

1. Verfahren zur Herstellung flüssiger Pflanzenauszüge, dadurch gekennzeichnet, daß als Extraktionsflüssigkeit eine wäßrige Lösung eines oder mehrerer nichtionischer Tenside eingesetzt wird.

2. Verfahren nach Anspruch 1, dadurch gekennzeichnet, daß die nichtionischen Tenside in wäßriger Lösung in einer Menge von 1 bis 10 Gew% eingesetzt werden.

3. Verfahren nach einem der Ansprüche 1 oder 2, dadurch gekennzeichnet, daß die nichtionischen Tenside in der wäßrigen Lösung in einer Menge von 5 bis 8 Gew% eingesetzt werden.

4. Verfahren nach einem der Ansprüche 1 bis 3, dadurch gekennzeichnet, daß wäßrige Lösungen von Mischungen verschiedener nichtionischer Tenside eingesetzt werden, wobei der Anteil des einzelnen Tensids zwischen 1 und 99 Gew% am Gesamttensidgehalt beträgt.

5. Verfahren nach einem der Ansprüche 1 bis 4, dadurch gekennzeichnet, daß als nichtionisches Tensid ein Umsetzungsprodukt aus 1 mol hydriertem Rizinusöl mit 40 bis 45 mol Ethylenoxid verwendet wird.

6. Verfahren nach einem der Ansprüche 1 bis 5, dadurch gekennzeichnet, daß die Extraktion bei einer Temperatur zwischen Raumtemperatur und einer solchen kurz unterhalb der Siedetemperatur der Extraktionsflüssigkeit vorgenommen wird.

## Claims

1. Process for the preparation of liquid plant extracts, characterized in that as the extraction liquid use is made of an aqueous solution of one or more nonionic surfactants.

2. Process according to claim 1, characterized in that the nonionic surfactants in aqueous solution are used in a quantity of 1 to 10 wt.%.

3. Process according to one of the claims 1 or 2, characterized in that the nonionic surfactants are used in the aqueous solution in a quantity of 5 to 8 wt. %.

4. Process according to one of the claims 1 to 3, characterized in that aqueous solutions of mixtures of different nonionic surfactants are used, the proportion of the single surfactant being between 1 and 99 wt.% of the total surfactant content.

5. Process according to one of the claims 1 to 4, characterized in that as the nonionic surfactant use is made of a reaction product of 1 mole of hydrogenated castor oil with 40 to 45 mole of ethylene oxide.

6. Process according to one of the claims 1 to 5, characterized in that extraction takes place at a temperature between ambient temperature and a temperature just below the boiling point of the extraction liquid.

## Revendications

1. Procédé pour la fabrication d'extraits végétaux liquides, caractérisé en ce que l'on utilise, comme liquide d'extraction, une solution aqueuse d'un ou de plusieurs tensides non ioniques.

2. Procédé selon la revendication 1, caractérisé en ce que les tensides non ioniques sont utilisés dans une solution aqueuse dans une quantité d'1 à 10 % en volume.

3. Procédé selon l'une des revendications 1 ou 2, caractérisé en ce que les tensides non ioniques sont utilisés dans la solution aqueuse dans une quantité de 5 à 8 % en volume.

4. Procédé selon l'une des revendications 1 à 3, caractérisé en ce que l'on utilise des solutions aqueuses de mélanges de différents tensides non ioniques, le pourcentage du tenside individuel étant d'1 à 99 % en poids du taux total de tensides.

5. Procédé selon l'une des revendications 1 à 4, caractérisé en ce que l'on utilise comme tenside non ionique un produit de transformation d' 1 mol d'huile de ricin hvdrogénée avec 40 à 45 mol d'oxyde d'éthylène.

6. Procédé selon l'une des revendications 1 à 5, caractérisé en ce que l'extraction est effectuée à une température entre la température ambiante et une température peu au-dessous de la température d'ébullition du liquide d'extraction.
